# EUROPÄISCHE PATENTSCHRIFT

(11) **EP 2 624 787 B1**
(45) Veröffentlichungstag und Bekanntmachung des Hinweises auf die Patenterteilung: **01.11.2017**
(21) Anmeldenummer: 11801572.6
(22) Anmeldetag: 05.10.2011
(51) Int. Cl.: A61F 2/30, A61F 2/44, A61F 2/48, A61L 27/04, A61L 27/14

(54) **MEDIZINISCHES IMPLANTAT FÜR DEN ZWISCHENWIRBELRAUM**
INTERVERTEBRAL MEDICAL IMPLANT
IMPLANT MÉDICAL POUR ESPACE INTERVERTÉBRAL

(30) Priorität: 05.10.2010 DE 102010041959
(43) Veröffentlichungstag der Anmeldung: 14.08.2013
(60) Teilanmeldung: 15001403.3
(73) Patentinhaber: Aces Ingenieurgesellschaft mbH, 70794 Filderstadt (DE)
(72) Erfinder: TRAUTWEIN, Frank, 70794 Filderstadt (DE); HEUER, Frank, 70794 Filderstadt (DE); FRANKE, Jörg, 39118 Magdeburg (DE); KOTHE, Ralph, D-22397 Hamburg (DE); LILJENQVIST, Ulf, 48147 Münster (DE); MATGÉ, Guy, L-8229 Mamer (LU); PUTZIER, Michael, 14532 Stahnsdorf (DE)
(74) Vertreter: Patentanwälte Magenbauer & Kollegen Partnerschaft mbB
(86) Internationale Anmeldenummer: PCT/DE2011/050040
(87) Internationale Veröffentlichungsnummer: WO 2012/052006

(56) Entgegenhaltungen:
- WO-A2-02/24122
- WO-A2-02/071921
- WO-A2-2006/091097
- US-A- 5 108 432
- US-A- 5 732 469
- US-A1- 2005 112 397
- US-A1- 2010 075 419
- US-B1- 6 200 347

## Beschreibung

Die Erfindung betrifft ein medizinisches Implantat, welches in den Zwischenraum zweier Wirbel eingeführt werden kann um die Wirbelsäule zu stabilisieren. Das erfindungsgemäße Implantat umfasst eine Vielzahl dünner Platten, wodurch vorteilhafte Eigenschaften bezüglich der Herstellung, Implantation und der Funktion im Körper realisiert werden können.

### Stand der Technik

Bei Erkrankungen der Wirbelsäule ist es oft erforderlich, eines oder mehrere Segmente zu stabilisieren. Hierfür gibt es eine Vielzahl von Zwischenwirbelimplantaten (auch "Cages" genannt) die eine knöcherne Fusion eines Wirbelsäulenabschnittes zum Ziel haben. Die Implantation solcher Cages kann zahlreiche Komplikationen nach sich ziehen. Beispielsweise können die Implantate aufgrund einer punktuellen Belastung oder kleinen Auflagefläche in die Deckplatte einbrechen, die erwünschte, knöcherne Fusion kann durch "Stress Shielding" ausbleiben, die Implantate können verrutschen oder die Stellung der Wirbelkörper zueinander kann nicht ausreichend korrigiert werden. Außerdem wird in der Regel eine sekundäre Abstützung (z.B. über ein Pedikelschrauben-Stab-System) benötigt, um das Segment soweit zu stabilisieren, dass eine Migration diese Implantate zuverlässig verhindert wird.

Aus WO9501763A1 1993 ist ein Wirbelsäulenimplantat bekannt, welches aus einem korbähnlichen Ring besteht und eine zentrale Öffnung für die Aufnahme von Knochenmaterial besitzt. In (EP1800627A2 2005) wurde beschrieben, dass ein Wirbelsäulenimplantat zur Kontaktflächenvergrößerung aus zwei oder mehreren Ringen bestehen kann, um so ein Einbrechen in die Wirbeldeckplatten zu vermindern. Aus WO0234171A2 2000 sind deformierbare Polymerstreifen bekannt, die in den Wirbelkörperzwischenraum eingebracht werden und zur Lastrichtung ausgerichtet werden können.

Aus den Anmeldungen US6767738B1 2004 und WO2008079953A2 2006 wird jeweils eine Anordnung von zwei oder drei rigide verbundenen Platten vorgeschlagen, die durch den Abstand der Platten einen Raum zur Einbringung von Knochen, Knochenersatzmaterial oder BMP erlauben. Die Platten erfüllen hier die Funktion einer Aufnahme um das mechanisch instabile Knochenmaterial in den Zwischenwirbelraum einzubringen und eine unerwünschte Verteilung im Bandscheibenraum bzw. im Körper zu verhindern. Aufgrund der gewünschten Funktion und der geringen Anzahl der Platten sind keine praktikablen Vorkehrungen vorgesehen die die Abstände der Zwischenräume einer größeren Anzahl von Platten bestimmt. Von Nachteil ist außerdem die erforderliche Gewinnung von Knochen zur Füllung des Zwischenraums, bzw. die Bereitstellung und Einbringung anderer Knochenersatzmaterialien.

Durch WO0045747A1 1998; WO0040177A1 1999 sind aus Knochenmaterial bestehende, geschichtete Anordnungen bekannt.

Aus der US 5,732,469 A ist ein Prothesensystem bekannt, bei dem zumindest Teilbereiche der Prothese aus aufeinander geschichteten, mit Durchbrüchen versehenen Materiallagen hergestellt sind, wobei die Dicke der Materiallagen 150 Mikrometer oder weniger angegeben ist und die einzelnen Materiallagen stoffschlüssig miteinander verbunden werden.

Die US 2005/112397 A1 offenbart ein Implantat, das als Schichtaufbau aus durchbrochenen Einzellagen aufgebaut ist und bei dem die Einzellagen mechanisch und/oder physikalisch, insbesondere stoffschlüssig durch Weich- oder Hartlöten, verbunden sind.

Der US 5,108,432 A sind ein künstliches Hüftgelenk und eine Schienbeinprothese zu entnehmen, die beide mit durchbrochenen Platten ausgerüstet werden können. Die Platten sind hierbei mit Durchbrüchen und Schlitzen versehen.

Aus der WO 2006/091097 A2) ist ein Schichtaufbau aus Einzellagen eines porösen Materials, insbesondere aus Metallfolien, bekannt, der zum Ersatz von menschlichen oder tierischen Knochen dienen soll, wobei die Einzellagen parallel zueinander angeordnet und direkt aufeinander geschichtet sind.

Die zuvor genannten Erfindungen bestehen aus oder erlauben die Einbringung von Knochen oder Knochenersatzmaterial in den Zwischenwirbelraum, ermöglichen jedoch weder die Korrektur der Wirbelstellung (Winkel und Versatz) noch bieten sie Lösungen zur gleichmäßigen Verteilung der Belastung auf die Deckplatten der Wirbelkörper. Aufgrund der nicht einstellbaren Höhe der Implantate sind während der Implantation teilweise hohe Einschlagkräfte und eine (Über)Distraktion der Endplatten erforderlich.

Aus WO0044319A1 1999 ist ein expandierbares Wirbelsäulenimplantat bekannt. Durch einen kleinen Bandscheibenzugang können zahlreiche zusammengefaltete Elemente eingebracht und durch eine Komprimierung so aufgestellt werden, dass sie die Wirbel distrahieren.

Aus(DE19816832C1 1998 und aus US2010010633A1 2008 sind Wirbelsäulenimplantate bekannt, welche mindestens eine Welle mit exzentrisch daran befestigten Scheiben besitzen. Die Welle ist verdrehbar, so dass die Scheiben herausgestellt werden und das Implantat an Bauhöhe gewinnt. Ein potentieller Nachteil dieser Ausgestaltung liegt in der Notwendigkeit einer steifen Verbindung zwischen der Welle und den Scheiben. Des Weiteren sind diese Wirbelsäulenimplantate auf die Distraktion beschränkt, wobei ein Verschieben der Wirbel zueinander aufgrund der drehbaren Welle und der entstehenden Reibung zwischen den Scheiben und den Deckplatten nicht vollständig ausgeschlossen werden kann. Die klinische Erfahrung zeigt, dass durch den weitgehend zylindrischen Querschnitt eine Vielzahl dieser Implantate in die Deckplatte einbrechen und diese teilweise schwer zu revidieren sind.

Aus US2010016968A1 2007 und WO2007048012A2 2005 sind Methoden und Implantate bekannt mit denen es möglich ist, eine Reposition eines abgeglittenen Wirbels durchzuführen. Dabei werden zwei implantierte Hälften gegeneinander verschoben bis die Endstellung erreicht ist. Hier basieren die Ansätze auf temporär implantierte Apparaturen die nach der finalen Versorgungen wieder entfernt werden müssen.

Neben den verschiedenen vorhergehend beschriebenen Unzulänglichkeiten beinhalten alle bisher bekannten Implantate keine oder lediglich einfache Verzahnungen an den Knochen-Kontaktflächen zur Erhöhung des Widerstandes gegen Migration. Die mechanische Erzeugung definierter Zahngeometrien mit einem Hinterschnitt ist aufgrund der bisher verwendeten Bauweisen und Fertigungsverfahren nicht bekannt. Bei der Verwendung von Knochen als Implantatwerkstoff verbietet die geringe Festigkeit des Werkstoffs die Ausgestaltung und Anwendung derartiger Zahngeometrien.

### Darstellung der Erfindung

### Technische Aufgabe

Die Aufgabe der vorliegenden Erfindung besteht in der Bereitstellung eines Implantats zur Stabilisierung der Wirbelsäule über den Zwischenwirbelraum. Wichtige Aufgaben des erfindungsgemäßen Implantats sind eine gleichmäßige Verteilung der Belastung über einen möglichst großen Teil der Kontaktfläche sowie die einfache aber sichere Verankerung in den Kontaktflächen ohne diese mechanisch zu schwächen. Das Erfindungsgemäße Implantat sollte außerdem kostengünstig in der Herstellung sein und die intra-operative Handhabung vereinfachen.

### Technische Lösung

Die Aufgabe wird für ein Wirbelsäulenimplantat mit den Merkmalen des Anspruchs 1 gelöst. Hierbei ist vorgesehen, dass das aus einer Vielzahl von Platten aufgebaute Wirbelsäulenimplantat, dessen Platten im Wesentlichen parallel angeordnet sind, mit einem Abstand zwischen den Platten zwischen 0,02mm und 3mm angeordnet sind.

Die Platten können knochenseitig eine filigrane Struktur besitzen, wobei durch Hinterschnitte in der Verzahnungsstruktur eine nochmalige Steigerung der Verankerungsfestigkeit in den Endplatten ermöglicht werden kann. Durch das gezielte Einbringen von weiteren Konturen bzw. Strukturen an und/oder in den Platten können unterschiedliche, optionale Funktionen des Implantats erzeugt und miteinander kombiniert werden. Es können zum Beispiel bestimmte Bereiche der Platten einen elastischen Charakter aufweisen und sich damit der Endplattenkontur anpassen bzw. einem "Stress-Shielding" entgegenwirken. Die Steifigkeit kann außerdem so eingestellt werden dass sie der von Knochen entspricht und dadurch einen Dehnungsreiz an das umgebende Gewebe gesendet wird. Im Verbund können die Platten mit einem Aktuator, dessen Verstellmechanismus, z.B. mit einer exzentrischen Welle, einem mechanischen, elektrischen oder auf formgedächtniseigenschaften basierten Mechanismus realisiert werden kann, so verbunden werden, dass sie Relativbewegungen vollziehen können, um beispielweise das Bandscheibenfach zu distrahieren oder zwei Wirbel relativ zueinander zu reponieren.

Erst die Verwendung von parallel angeordneten Platten dünner Wandstärke erlaubt die kostengünstige Fertigung der notwendigen, teilweise komplexen Strukturen. Die Konturen können hierzu bevorzugt durch ein photo-chemisches Ätzverfahren hergestellt oder durch energiereiche Strahlung (z.B. Laserstrahlen, Elektronenstrahlen) bzw. durch einen Wasserstrahl aus den Platten geschnitten werden. Darüber hinaus sind die Konturen ganz oder teilweise durch Drahterodieren oder Spritzgießen (aus Metall- oder Kunststoff) herstellbar.

### Vorteilhafte Wirkungen

Vorteilhaft bei dem erfindungsgemäßen Implantat ist der durch den besonderen Verzahnungsaufbau mögliche sehr hohe Widerstand gegen Migration und Einbrechen in die Grund- und Deckplatten. Dadurch ergibt sich eine sehr hohe Primärstabilität, wodurch eine zusätzliche Stabilisierung über ventrale Platten oder dorsale Stabsysteme bei einem Teil der Patienten überflüssig wird. Durch die Vielzahl der sich ergebenden Kanäle bzw. Gitterstrukturen und einer elastischen Lagerung der Verzahnung bzw. Einstellung der Implantatsteifigkeit über geeignete Aussparungen wird außerdem ein großer Stimulus zur Bildung von Knochen erzeugt ohne dass das Implantat mit zusätzlichem Knochen oder Knochenersatzmaterial befüllt werden muss. Durch die verschiedenen Verstellmöglichkeiten wird einerseits eine optimale Anpassung an die Patientenanatomie erreicht, andererseits wird die Implantation bei den Ausführungen vereinfacht die nach der Einbringung distrahiert oder im Winkel eingestellt werden können. Eine weitere Variante stellt zudem eine Möglichkeit der einfachen Korrektur der AP-Ausrichtung der Wirbelkörper über eine im Implantat integrierte Funktionalität vor.

### Kurze Beschreibung der Zeichnungen

- Fig. 1: zeigt die implantierbare Erfindung zur Fixierung eines Wirbels.
- Fig. 2: beschreibt die erste Ausführungsvariante der Erfindung.
- Fig. 3: beschreibt eine zweite Ausgestaltungsversion mit ineinander steckbaren Platten.
- Fig. 4: zeigt die Implementierung von Verankerungselementen zur gleichmäßigen Lastverteilung der Platten.
- Fig. 5: illustriert den Aufbau eines nicht zur Erfindung gehörenden distrahierbaren Implantats.
- Fig. 6: schildert eine Modifikation der nicht zur Erfindung gehörenden, in Fig. 5 gezeigten Variante.
- Fig. 7: zeigt den zu Fig. 6 gehörigen Implantataufbau der nicht zur Erfindung gehörenden Variante.
- Fig. 8: beschreibt den Aufbau einer weiteren Ausgestaltungsvariante.
- Fig. 9: beschreibt eine weitere Ausgestaltungsvariante, des in Fig. 6 beschriebenen Implantats.
- Fig. 10: zeigt eine primärstabile Variante eines Bandscheibenimplantats.
- Fig. 11: zeigt ein Implantat für den postero-lateralen Zugang und die Realisierung einer ungleichmäßigen Höhenzunahme zur über die Implantatbreite.
- Fig. 12 und 13: zeigen zwei beispielhafte Methoden, wie die Erfindung implantiert werden kann.
- Fig. 14: zeigt wie das nicht zur Erfindung gehörende Wirbelsäulenimplantat aufgebaut ist, um einen Wirbel zu reponieren.
- Fig. 15: erläutert die mechanische Umsetzung der Korrekturbewegung des in Fig. 14 gezeigten, nicht zur Erfindung gehörenden Implantats.
- Fig. 16: illustriert die Arbeitsschritte, die bei der Implantation der zuvor genannten Ausgestaltungsform zu berücksichtigen sind.
- Fig. 17: beschreibt eine nicht zur Erfindung gehörende, alternative Ausführungsform zu der in Fig. 14 gezeigten Ausgestaltungsvariante.
- Fig. 18: zeigt den Implantatvorgang der 8b Variante.

### Weg(e) zur Ausführung: der Erfindung

Die technischen Lösungen sind nachfolgend oft beispielhaft beschrieben. Dies soll als Mittel zur Erläuterung des zugrundeliegenden Gedankens aufgefasst und nicht als auf die jeweilige konkrete Darstellung beschränkt verstanden werden. Die hier im Einzelnen beschriebenen Ausführungsformen bilden eine kombinatorische Grundlage der Erfindung, das heißt die Ausführungsformen können einzeln oder ein Kombination miteinander ausgeführt werden.

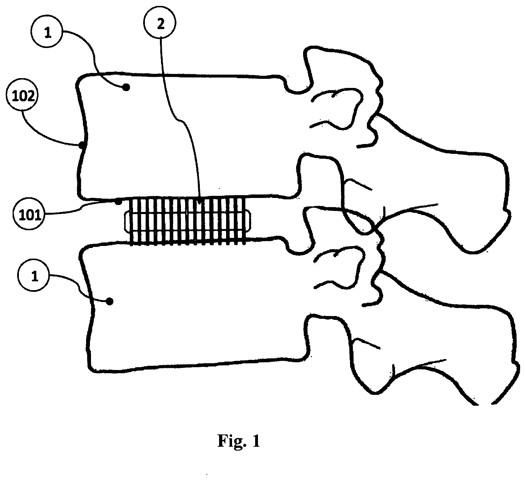

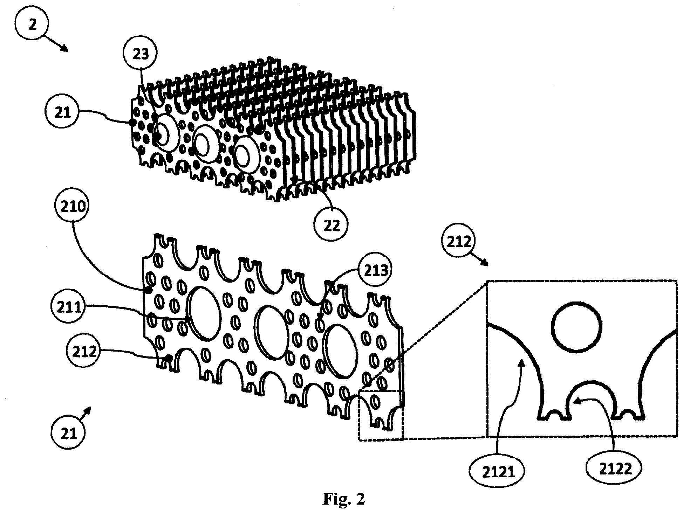

Das erfindungsgemäße Implantat (2) wird in den Zwischenraum zweier Wirbel (1) eingeführt um die Wirbelsäule zu stabilisieren (Fig. 1). Dabei entsteht mindestens eine Kontaktfläche zwischen Implantat und den Deckplatten (101) der angrenzenden Wirbel (1). Das Wirbelsäulenimplantat (2) besteht aus einer Vielzahl von Platten (21), die zusammen mit ihren Zwischenräumen (22) mit Hilfe mindestens eines Verbindungselements (23) miteinander verbunden sind (Fig. 2). Die Platten (21, 210) weisen eine Wandstärke von 0,05 bis, 3mm, insbesondere zwischen 0,2 bis 1,5 mm auf. Die Breite der Zwischenräume (22) orientiert sich an der Größe der Trabekelstruktur des Knochens und beträgt ebenfalls zwischen 0,05 und 3mm, insbesondere zwischen 0,2 und 1,5mm.

Die Platten können beispielsweise durch Spritzgießen aus Metall (Metal Injection Molding), Kunststoff oder aus anderen biokompatiblen bzw. bio-resorbierbaren Werkstoffen, oder einer Kombination dieser Werkstoffe hergestellt werden. Andere besonders geeignete Fertigungsverfahren für die Herstellung der Platten sind das Ausschneiden mit Hilfe energiereicher Strahlen (Elektronenstrahl, LASER, Wasserstrahl) oder das Herausätzen über ein photochemisches Ätzverfahren aus Metallbändern, Blechen oder Platten (Photo-Etching). Die genannten Herstellungsverfahren bieten eine Vielzahl von Ausgestaltungsmöglichkeiten der Platten und erlauben die kostengünstige Integration von Funktionen in das Implantat, die durch die bekannten ein- oder zweiteiligen, im Wesentlichen monolithischen Ausführungsformen nicht realisiert werden können. So ist es zum Beispiel auf einfache Weise möglich, Strukturen, Profile, Führungen, Öffnungen oder Verzahnungen (212) mit unterschiedlicher Austragungstiefe in die Platten zu integrieren, Verzahnungen mit Hinterschnitt herzustellen oder Zähne jeweils versetzt zur nächsten Platte anzuordnen. So kann die Knochen-Auflagefläche z.B. durch eine Vielzahl kleiner Verzweigungen (2121) eine "Mikropenetration" der Deckplatte ermöglichen (101), wobei die Verzweigungen einen Hinterschnitt (2122) aufweisen können und dadurch bereits nach einer kurzen Einheilungsphase eine primärstabile Verankerung im Knochen ermöglichen und die Verankerungsfestigkeit insbesondere unter Zugbelastung deutlich erhöht wird. Durch den Plattenaufbau und die damit möglichen Fertigungsverfahren können semi-permeable Poren, Kapillaren oder Öffnungen (213) hergestellt werden, welche den Aufbau eines osmotischen Drucks im Inneren des Implantats hervorrufen, und dieser wiederum zur Stoffwechselförderung und Zelldifferenzierung genutzt werden kann. Um die Plattenoberfläche osteokonduktiv oder/und osteoinduktiv zu gestalten, können die Platten mit einem geeigneten Material beschichtet sein oder in einer definierten Rauigkeit hergestellt sein. Außerdem kann der Werkstoff aller oder einzelner Platten so gewählt werden, dass diese über die Zeit resorbierbar sind. Zur definierten Resorption über einen langen Zeitraum bietet es sich an, die Platten mit unterschiedlicher Dicke bzw. aus unterschiedlichen Materialien mit unterschiedlicher Resorptionsrate herzustellen.

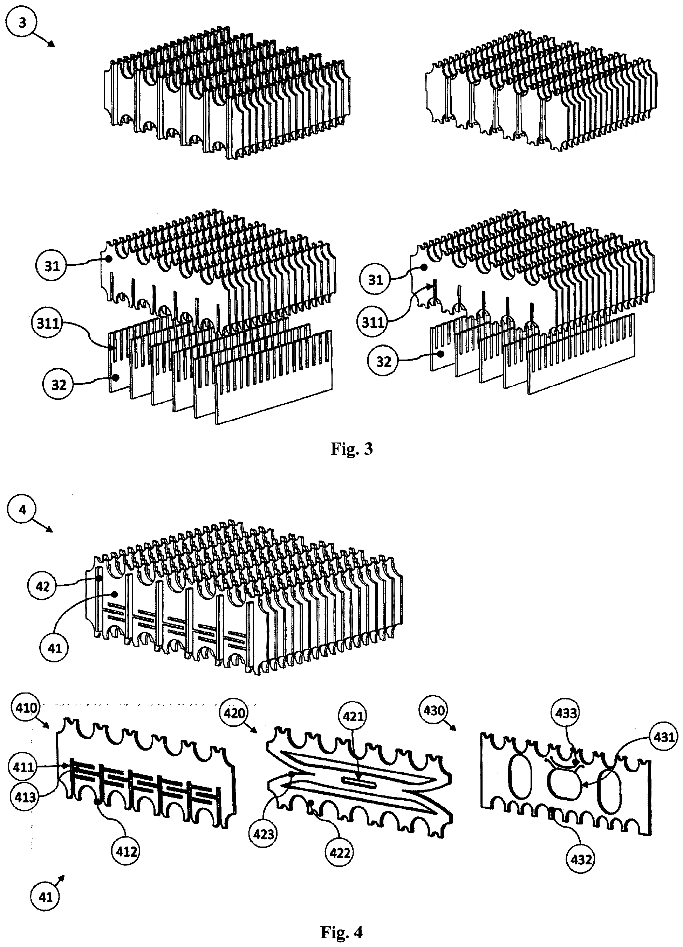

In einer weiteren Ausführungsvariante (3) können die Platten so ausgestaltet sein, dass sie ineinander steckbar sind (Fig. 3). Dies kann dadurch erreicht werden, indem die Platten (31) und/oder (32) schlitzförmige Verbindungsöffnungen (311) aufweisen, die jeweils miteinander kompatibel sind und eine formschlüssige Verbindung ermöglichen. Der Abstand der Schlitze (311) in der Verbindungsplatte (32) definiert dabei die Teilung und damit den Abstand des Zwischenraumes (22).

In einer dritten Ausgestaltungsform der Erfindung (4) ist beispielhaft die dynamische Ausgestaltung der Platten (41) in Fig. 4 gezeigt. Damit ist das Implantat in der Lage, sich der Deckplattenkontur (101) anzupassen und damit eine gleichmäßige Kraftverteilung auf die Deckplatte zu ermöglichen, ohne einen Deckplatteneinbruch infolge lokaler Kraft- und Spannungskonzentrationen zu provozieren. Die Platten (410) können dabei so gestaltet sein, dass jede einzelne die Knochen-Kontaktfläche bildende Haken- oder Zahnstruktur (412) beispielsweise durch einen mäanderförmigen Konturausschnitt (413) federnd gelagert ist.

Eine weitere Ausgestaltungsvariante ist in Fig. 4 (420) gezeigt. Hier wird die federnde Lagerung der Knochen-Kontaktfläche über mehr als einem Hakenelement (422) ermöglicht, indem ein Konturausschnitt (423) größer ausgestaltet ist. Des Weiteren kann eine federnd gelagerte Verbindung der Platten (430) dazu verwendet werden, eine Elastizität oder Steifigkeitsreduktion des gesamten Implantats zu erzielen. Dies kann erreicht werden indem die Verbindungsöffnungen (431) einen benachbarten Konturausschnitt (433) erhalten, welcher eine elastische Nachgiebigkeit im Bereich der Lastübertragungsfläche der Verbindungs- bzw. Einstellöffnung (431) ermöglichen.

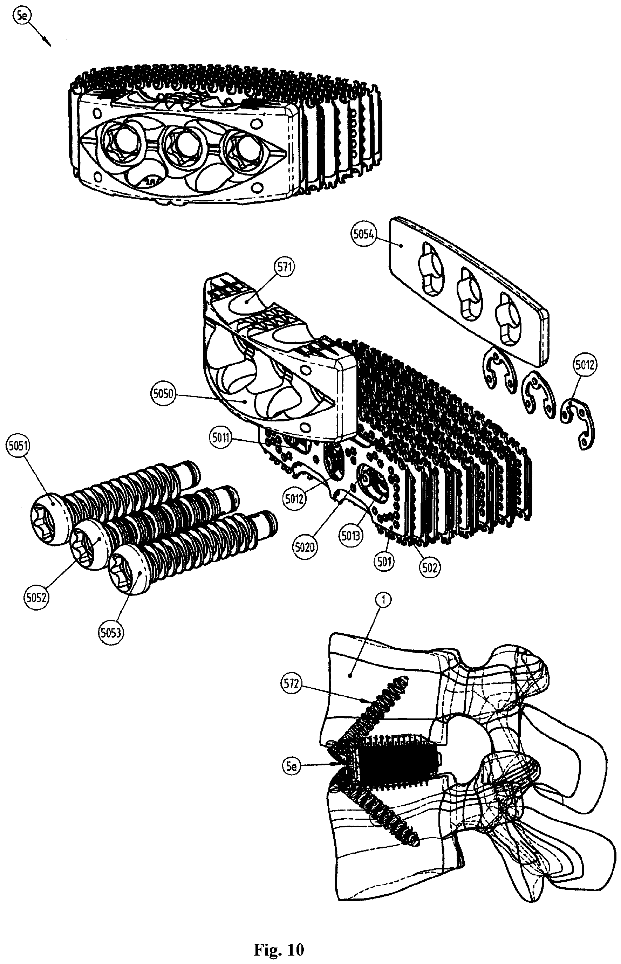

Fig. 4 zeigt außerdem verschiedene Ausschnittformen (411, 421, 431) zur Aufnahme von weitgehend rechtwinklig zu den Platten (41) angeordneten Verbindungselementen (42, 23, 5051, 5052, 5053 usw.). Die Verbindungselemente können als Schraube, Stift, Rohr oder Achse, mit rundem, ovalem, rechteckigem oder Polygon-Querschnitt ausgeführt sein. Verbindungselemente in Plattenform (32, 42) eignen sich zur gezielten Einstellung eines Abstands des Zwischenraumes (22) zwischen den Platten. Der Abstand der Platten ist dabei bevorzugt so zu wählen, dass er mit der Strukturgröße der Knochentrabekel kompatibel ist, also von ca. 0,02 bis 3mm und insbesondere zwischen 0,2 und 1,5mm. Eine alternative Möglichkeit zur Einstellung des Zwischenraumabstands (22) besteht in der Abstufung des Querschnitts von Verbindungselement (42, 5051, 5052, 5053 usw.) entlang seiner Achse und der Verbindungsöffnung (421, 431) der Platten (41). Bei einer großen Plattenanzahl ist jedoch insbesondere die Verwendung von Scheiben (5020, Fig. 10), Ringen oder ähnlichen Elementen mit einer Dicke die dem gewünschten Zwischenraumabstand (22) entspricht vorteilhaft. Hierzu werden die Abstandsscheiben (5020) jeweils zwischen den Platten (41) auf den Verbindungselementen (23, 5051, 5052, 5053 usw.) angeordnet. Schließlich ist eine zwischen den Platten (41) räumlich versetzte Prägung der selbigen dazu geeignet, einen definierten Abstand (22) zwischen den Platten einzustellen.

Das Implantat kann derart gestaltet sein, dass die Montage von Platten (21), Verbindungselement (23 bzw. 32) und ggf. separaten Abstandsscheiben- oder Ringen (5020) patientenspezifisch während der Operation erfolgt. Dies kann dadurch erzielt werden, indem ein Instrument in das Bandscheibenfach eingebracht wird, welches einen einstellbaren oder deformierbaren Bereich besitzt mit dem sich die Deckplattenkontur erfassen oder temporär abformen lässt. Das Instrument wird wieder aus dem Bandscheibenfach entfernt und das Wirbelsäulenimplantat kann während der Operation aus einzelnen Platten (21) unterschiedlicher Höhe und Breite der Deckplattenkontur entsprechend zusammengebaut werden.

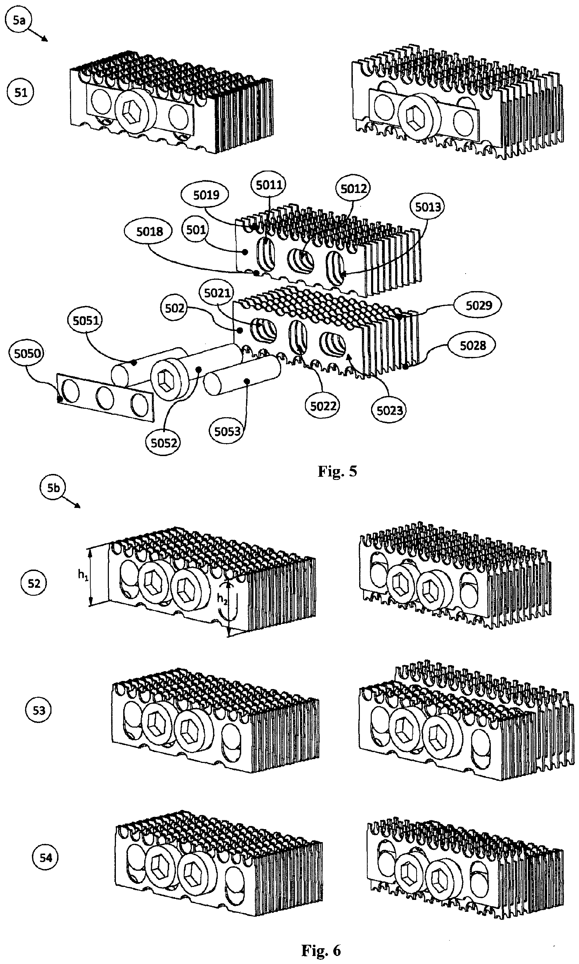

In der Figur 5 wird eine nicht zur Erfindung gehörende Ausführungsvariante (5a) eines Implantats mit verstellbarer Höhe vorgestellt. Beim Einführen des Wirbelsäulenimplantats (5a) in den Zwischenwirbelraum weist der Plattenverbund, bestehend aus oberen (501) und unteren (502) Platten zunächst eine minimale Höhe auf, wobei die Verzahnungsstrukturen (5019 und 5028) durch abgeflachte Profile (5018 und 5029) überdeckt sind und damit das Einführen des Implantats erleichtern. Erst nach der Höhenverstellung, bei der die Plattenverbünde (501 und 502) gegenseitig verschoben werden, ragen die Verzahnungsstrukturen (5019 und 5028) über die abgeflachten Profile (5018, 5029) hinaus in die Deckplatten (101) und dienen dort zur Verankerung. Die Platten (501 und 502) sind so gestaltet, dass sie diverse Verbindungsöffnungen besitzen. Dabei wird zwischen den Öffnungen für die Lagerung (5011, 5013, 5021 und 5023) und den Öffnungen für die Einleitung der Relativbewegung (5012 und 5022) der Platten unterschieden. Die Orientierung der Öffnungen (5011-5013) ist dabei zur Erzielung einer Relativbewegung zwischen den Plattenverbünden (501 und 502) von der Orientierung der Öffnungen (5021-5023) verschieden, in einer bevorzugten Ausführung um 90° gedreht. Gelagert werden die Platten (501 und 502) in diesem Ausführungsbeispiel über zwei Achsen (5051 und 5053). Die Relativbewegung der Platten erfolgt über einen Aktuator, welcher in diesem Beispiel über eine Exzenterwelle (5052) und ein damit verbundenes Instrument zur Übertragung einer Drehbewegung realisiert ist. Durch Drehung der Exzenterwelle (5052) bewegt sich der Ausschnitt (5012) und damit der Plattenverbund (501) analog zur Stellung des Kurbelradius der Exzenterwelle (5052). Eine senkrecht zur Welle angeordnete Platte (5050) oder ein das Implantat umschließender Rahmen hält dabei die Verbindungselemente (5051, 5053), die Exzenterwelle (5052) und indirekt über die Lager (5051, 5053) den unteren Plattenverbund (502) in einer konstanten Position. In den weiteren Ausführungen wird der Rahmen (5050) nicht weiter dargestellt um einen Einblick auf den inneren Aufbau zu gestatten.

Nachteilig bei der in Fig. 5 dargestellten Variante ist die Kippneigung des oberen Plattenverbunds (501) über die Exzenterwelle (5052). Die Ausgestaltungsform (5a) kann deshalb dahingehend erweitert werden (Fig. 6, 5b), dass zwei Exzenterwellen (5052) verwendet werden. Dies verhindert die Kippneigung der Plattenverbünde und ermöglicht die separate Höheneinstellung h1 und h2. Durch Gestaltung der die Relativbewegung beeinflussenden Ausschnitte (5012) bzw. die Position und Länge des Abschnitts der Exzentrizität auf der Exzenterwelle (5052) ist es möglich, nur Teile des jeweiligen Plattenverbunds zu verstellen. So ist es beispielsweise möglich, beide Bereiche (52), nur den hinteren (53) oder nur den vorderen Bereich (54) bei Verdrehung der Exzenterwellen (5052, 5053) in der Verstellhöhe zu variieren.

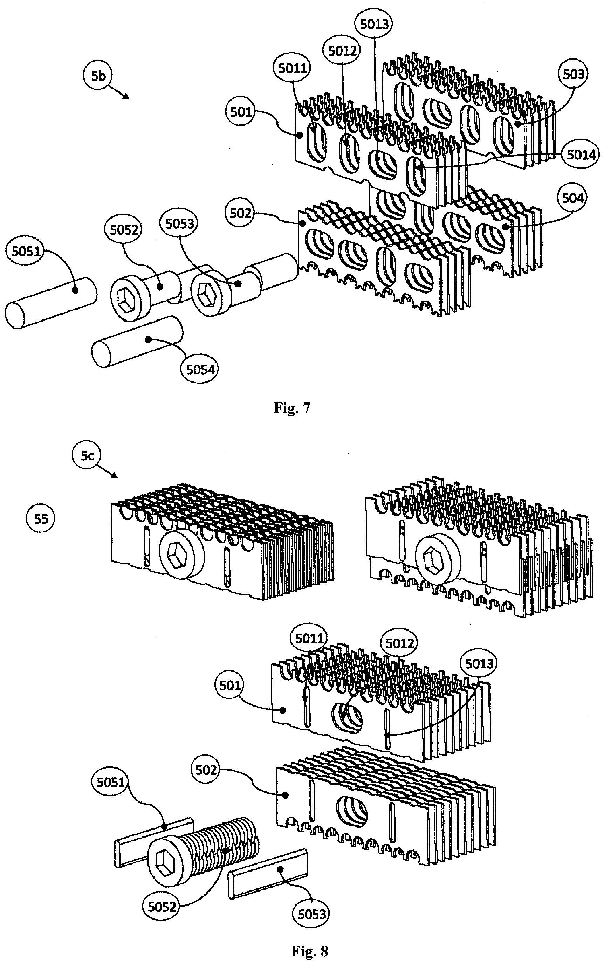

Fig. 7 beschreibt eine nicht zur Erfindung gehörende Variante mit vier verschiedenen Plattenverbünden (501, 502, 503 und 504), die sich in ihren Verbindungsöffnungen (z.B. 5011, 5012, 5013 und 5014) unterscheiden. Der Unterschied kann beispielsweise durch eine alternative Orientierung der Verbindungsöffnungen oder deren Abmessungen erreicht werden. Außerdem sind die Exzenterwellen mit je zwei Abschnitten dargestellt die einen unterschiedlichen Kurbelradius aufweisen.

Die Ausgestaltung der Höhenverstellung kann außerdem so gestaltet sein (5c, Fig. 8), dass die Platten (501 und 502) bezüglich der Ausschnitte identisch sind und die Exzenterwelle (5052) eine Vielzahl von Einzelexzentern mit gleichem oder unterschiedlichem Kurbelradius besitzt. Damit ist es möglich entlang der Exzenterwelle (5052) unterschiedliche Relativbewegungen zu erzielen. Zur Verringerung der vorher erwähnten Kippneigung bei nur einer Exzenterwelle können außerdem überwiegend rechteckige Verbindungselemente (5051 und 5053) verwendet werden.

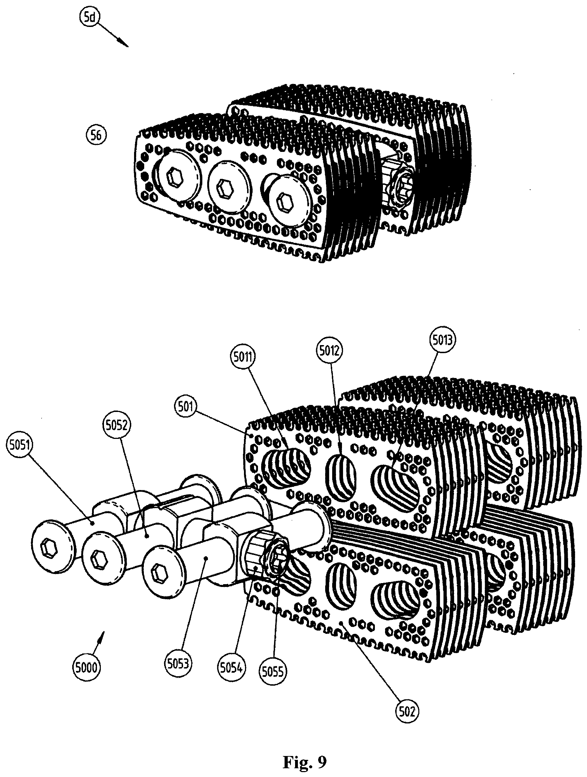

In einer weiteren nicht zur Erfindung gehörenden Ausgestaltungsform (5d, Fig. 9) ist der Verstellmechanismus durch Ausschnitte (5011, 5013) in den Platten (501, 502) und einen darin eingreifenden Aktuator (5000) realisiert, wobei der Aktuator wenigstens zwei Achsen (5051 und 5053), Wellen, Bolzen, Schrauben, Stifte, Kulissensteine, Keile oder ähnliche Verbindungselemente beinhaltet, deren Abstand zueinander einstellbar ist. Durch die Abstandsänderung der Achsen erfolgt eine Höhen- oder Winkeländerung des Implantats, wobei die Divergenz der korrelierenden Ausschnitte (5011, 5013) in Verbindung mit der Achsenabstandsänderung die resultierende Höhenänderung des Implantats bestimmt. Die in Fig. 9 dargestellte mittlere Achse (5052) dient der Lagerung bzw. Fixierung des Aktuators (5000) und ist in den mittleren Aussparungen (5012) der Platten gelagert. Die Achsen können dann durch ein Instrument, welches beispielsweise auf eine Zahnstange mit Ritzel, eine rastende oder klemmbare Verstelleinrichtung oder eine Gewindespindel (5054 oder 5055) einwirkt in ihrem Abstand verstellt werden. Weitere Verstellmöglichkeiten bestehen durch Einsatz einer elektrisch oder piezoelektrisch betriebenen Verstelleinrichtung oder durch eine auf einem Formgedächtnismaterial beruhenden Längenänderung innerhalb des Aktuators (5000).

Sollen die Kontaktflächen mit den Wirbelendplatten nicht parallel (bzw. undefiniert) sondern mit einem bestimmten Winkel eingestellt werden, ist dies durch die gleichzeitige Verschiebung beider Achsen in dieselbe Richtung möglich. Dies kann beispielsweise durch eine Spindellagerung erreicht werden, deren Position über ein Gewinde relativ zum Plattenverbund bzw. des umgebenden Rahmens einstellbar ist. Das Verschieben der Achsen (5051, 5052 und 5053) zueinander kann, wie gezeigt, mit einer Spindel oder anderen linearen Antrieben realisiert werden.

In der nicht zur Erfindung gehörenden Ausgestaltungsform (5e) ist eine weitere Version des Bandscheibenimplantats dargestellt (Fig. 10). Die Variante (5e) besteht aus einer Vielzahl von Platten, welche abwechselnd zur superioren und inferioren Deckplatte ausgerichtet sind (501 und 502). Ein Merkmal dieser Ausführung ist die Kombination mit einer Aufnahme (5050), welche mindestens eine schräg verlaufende Öffnungen (571) besitzt. Durch diese Öffnung (571) können den Wirbelkörper penetrierende Knochenanker (572) geführt und mit dem Knochen (1) verbunden werden. Diese Ausgestaltungsform (5e) bietet dadurch eine nochmals erhöhte Primärstabilität. Des Weiteren besitzt diese Ausführungsform (5e) drei Exzenterwellen (5051, 5052 und 5053) die anterior (5050) und posterior (5054) gelagert und durch Sicherungsringe (5012) axial positioniert sind. In Abhängigkeit der Nockenform und platzierung können verschiedene biomechanisch wichtige Eigenschaften eingestellt werden. Beispielsweise bietet die hier gezeigte Ausgestaltungsform (5e) durch entsprechende Wahl der Ausschnitte (5011, 5012, 5013) die Möglichkeit das Implantat sowohl seitlich als auch in der Höhe zu expandieren.

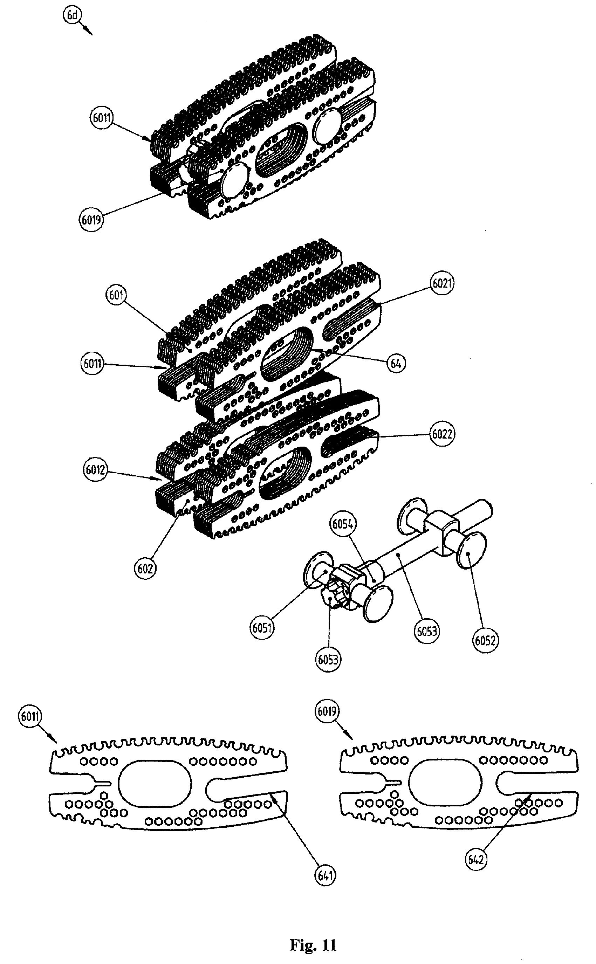

Version (6) zeigt eine für den postero-lateralen Zugang optimierte, nicht zur Erfindung gehörende Ausgestaltungsform (Fig. 11). Das hier gezeigte Implantat kann einen (oder mehrere) Ausschnitte (64) für die Anlagerung von Knochenmaterial besitzen. Zur besseren Distraktion des Bandscheibenfaches kann der anteriore Aufrichtwinkel des Implantats der vorderen Führungsnuten (6012, 6022) der Platten (601, 602) von medial nach lateral angepasst werden. Dabei wird der Winkel (642) der vorderen Nut (6021, 6022) bei jeder Platte geändert, z.B. von lateral (6011) nach medial (6019), so dass die Höhenzunahme ein schräg verlaufendes Profil aufweist und damit optimal an die Geometrie der Deckplatten angepasst ist.

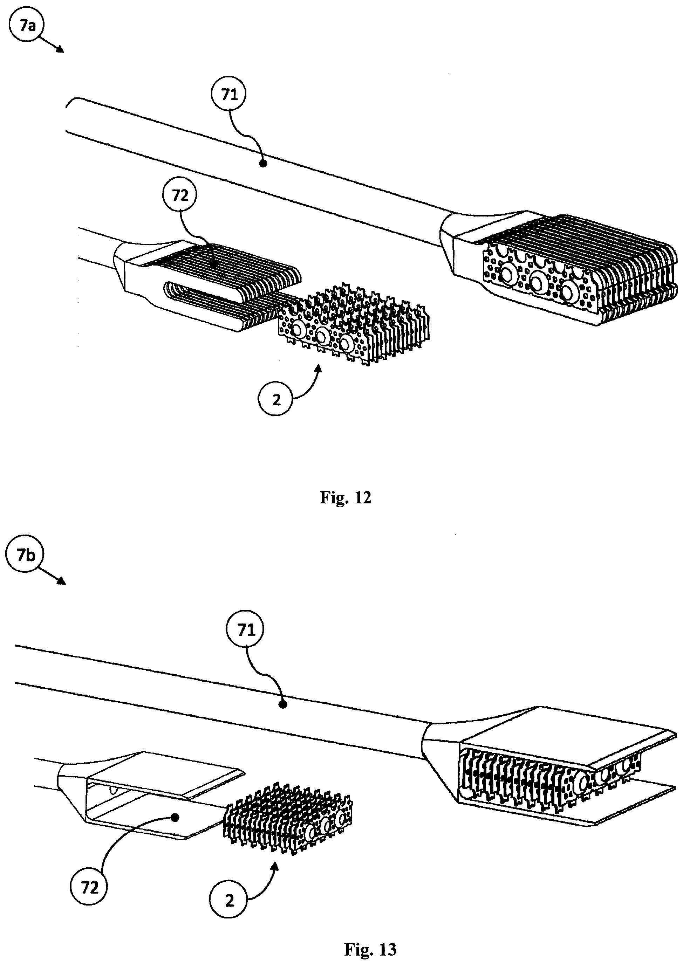

Für die Implantation des erfindungsgemäßen Implantats (2) werden zwei Implantationsinstrumente (7a und 7b) beispielhaft gezeigt (Fig. 12 und Fig. 13). Zum Einen kann das Instrument (7a) so ausgestaltet sein, dass es aus einem Schaft (71) mit einem distalen Ende (72) besteht. Die Platten des Implantats werden vom distalen Ende (72) formschlüssig umgeben, so dass die Platten mit den Hakenelementen durch das Instrument vor einem unbeabsichtigten Verbiegen beim Einführen geschützt sind, ohne die Höhe des Implantats durch die Wandstärke des Instruments zu vergrößern. In der Ausführung (7b) wird ein Instrument für ein anders orientiert einzubringendes Implantat gezeigt welches die gleichen präventiven Funktionen der Ausführung 7a erfüllt, jedoch die Gesamthöhe während der Implantation geringfügig vergrößert. Durch den Schaft (71) kann das Implantat mit dem Instrument (z.B. über eine durchgehende Gewindestange) verbunden werden, so dass das Implantat sicher gehalten wird und ggf. auch explantiert werden kann.

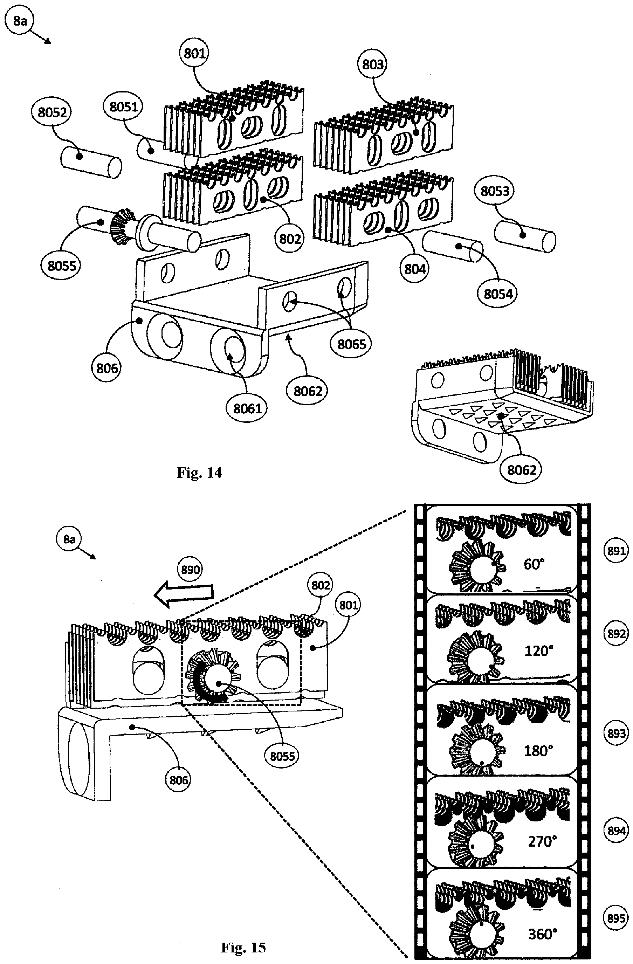

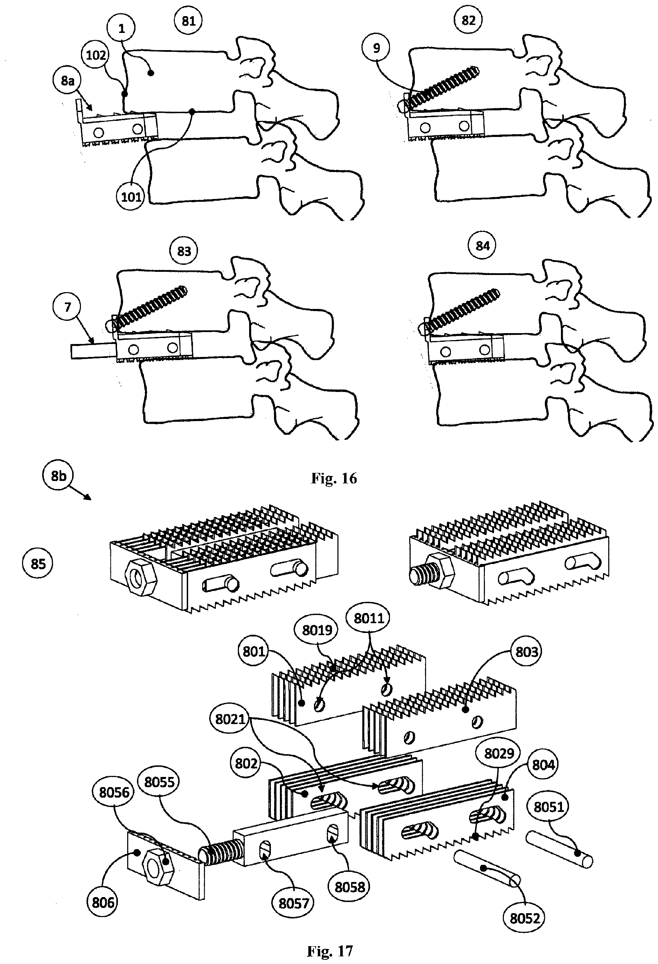

In einer weiteren nicht zur Erfindung gehörenden Ausführungsform (8a) wird die Realisierung eines Repositionsmechanismus der Erfindung beschrieben (Fig. 14). Das Wirbelsäulenimplantat (8a) besteht aus mindestens einem Plattenverbund (801, 802, 803 und 804), einer Halterung (806), verschiedenen Verbindungselementen (8051, 8052, 8053 und 8054) und wenigstens einer Exzenterwelle (8055). Die Verbindungselemente (8051, 8052, 8053 und 8054) sind an den Lagerstellen (8065) mit der Halterung (806) verbunden. Des Weiteren kann die Halterung (806) geeignete Elemente (8062) zur Verankerung in der Deckplatte besitzen. Die Komponenten sind so angeordnet, dass bei Rotation der Exzenterwelle (8055) eine kombinierte Hub- und Translationsbewegung im Plattenverbund stattfindet (Fig. 15). So ist es beispielsweise möglich, dass die Rotation (891-895) der exzentrischen Welle (8055) Peristaltikbewegungen zwischen den Platten (801 und 802) erzeugt, die in der Summe eine lineare Bewegung oder Translation (890) auf der Verzahnungsfläche des Implantats (8a) ergeben. Diese mechanische Umsetzung kann dazu verwendet werden, dass ein fehlgestellter Wirbel reponiert und damit bezüglich seiner AP-Lage korrigiert wird. In Fig. 16 ist ein solcher Repositionsvorgang illustriert. Dazu wird das Implantat (8a) in den Wirbelzwischenraum eingeführt (81). Das Ende mit der Halterung wird dabei an einer anterioren Wirbelkante (102) fixiert (82); hier beispielhaft mit einer Knochenschraube (9) gezeigt. Anschließend wird mit einem Instrument (7) eine rotatorische Bewegung auf die Exzenterwelle (8055) übertragen (83). Aufgrund der Rotation der Exzenterwelle (8055) übt das Wirbelsäulenimplantat eine Translationsbewegung (890) auf die Deckplatte des unteren Wirbels in AP-Richtung aus, wodurch der Wirbel positioniert bzw. seine Lage korrigiert werden kann (84).

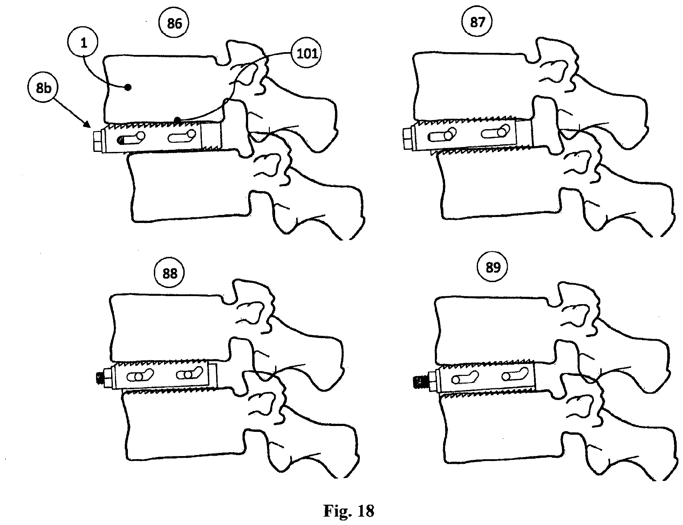

Eine modifizierte Ausführung des nicht zur Erfindung gehörenden Repositionsmechanismus (8b) ist in Fig. 17 dargestellt. Das Wirbelsäulenimplantat (8b) besteht aus mindestens einem Plattenverbund (801, 802, 803 und 804), einer Halterung (806), verschiedenen Verbindungselementen (8051, 8052) und wenigstens einer Spindel (8055). Die Verbindungselemente (8051, 8052) sind an den Lagerstellen (8057 und 8058) mit der Spindel (8055) verbunden. Die Spindel (8055) ist zum Rahmen (806) mit einer Spindelmutter (8056) verbunden. Des Weiteren weisen die Platten zum Knochen hin Strukturen auf, die eine Verankerung der Deckplatten (101) in der Repositionsrichtung begünstigen (8019 und 8029). Die Spindel wird mit Hilfe einer Spindelmutter (8056) verschoben, dadurch wird ein Teil des Plattenverbundes (801 und 803) transportiert (85). Aufgrund des Verlaufs der Bohrungen und Schlitze (8011 und 8021) für die Lagerwellen (8051 und 8052) kann das Implantat zuerst in der Höhe distrahiert werden (87) (Fig. 18). Nach weiterem Verfahren der Spindel findet eine Translation statt (88), die für eine Translation und damit Reponierung (89) sorgt.

### Zusammenfassung

Die Fusion zweier Wirbel ist eine der am häufigsten durchgeführten Eingriffe der Wirbelsäulenchirurgie. Die vorgeschlagene Erfindung betrifft ein Wirbelsäulenimplantat, welches aus einer Vielzahl von parallel angeordneten Platten aufgebaut ist. Durch das gezielte Einbringen von Konturen in den Platten können biomechanisch vorteilhafte Funktionen und Einstellmöglichkeiten erzeugt werden, die auf andere Weise nicht oder nicht kostengünstig realisierbar sind. So kann sich das erfindungsgemäße Implantat durch eine Elastizität der Verankerungselemente an die knöchernen Deckplatten anpassen, um so für eine gleichmäßige Kraftverteilung zu sorgen und damit der Gefahr der Einsinterung bzw. des Deckplatteneinbruchs entgegenzuwirken. Durch den Plattenaufbau können Fertigungsverfahren verwendet werden, die die Herstellung von Haken-ähnlichen Hinterschnitt-Konturen ermöglichen und damit eine hervorragende Verankerung im Knochen erlauben ohne diesen zu schädigen. Bei einer nicht zur Erfindung gehörenden Variante können die Platten über einen Aktuator derart miteinander verbunden werden, dass sich diese in der Höhe und/oder im Winkel verstellen lassen und somit eine Aufrichtung und phisiologische Einstellung des Bandscheibenfachs erlauben. Die Aufrichtung kann über die Länge des Implantats unterschiedlich sein, so dass auch eine Winkeleinstellung des Segments ermöglicht wird. Werden die Platten über eine Exzenterwelle miteinander verbunden, so kann die auf den Plattenverbund ausgeübte kombinierte Hub- und Translationsbewegung außerdem zur translatorischen Repositionierung zweier Wirbel verwendet werden.

## Patentansprüche

1. Medizinisches Implantat (2) zur Platzierung zwischen Knochen, insbesondere zwischen Wirbelkörper, wobei das Implantat aus einer Vielzahl (>3) von dünnen Platten (21, 31) besteht, eine Materialstärke der Platten zwischen 0,02 und 3mm beträgt, die Platten mit Aussparungen (213) versehen sind, die dazu geeignet sind von Körperzellen besiedelt und durchbaut zu werden, die Platten durch mindestens ein Verbindungselement (23, 32) verbunden sind, die Platten in wenigstens einer Richtung weitgehend parallel zueinander angeordnet sind, **dadurch gekennzeichnet, dass** ein Abstand (22) zwischen den Platten zwischen 0,02mm und 3mm beträgt.

2. Medizinisches Implantat nach Anspruch 1, **dadurch gekennzeichnet, dass** das Verbindungselement (23, 5051, 5053) als Schraube, Stift oder Rohr ausgeführt ist.

3. Medizinisches Implantat gemäß einem der vorherigen Ansprüche, **dadurch gekennzeichnet, dass** ein Abstand (22) zwischen den Platten (21) durch Ringe (5020) oder Scheiben, welche sich auf den Verbindungselementen (23, 5051, 5052, 5053) befinden, definiert ist.

4. Medizinisches Implantat gemäß einem der vorherigen Ansprüche, **dadurch gekennzeichnet, dass** der Abstand (22) der Platten (21) durch versetzte Prägungen oder Ausformungen definiert ist.

5. Medizinisches Implantat gemäß einem der vorherigen Ansprüche, **dadurch gekennzeichnet, dass** das Verbindungselement (32) in Gestalt eines Streifens oder Platte ausgeführt ist, das Verbindungselement (32) über Schlitze (311) verfügt und der Abstand der Schlitze (311) den Abstand (22) der Platten (31) definiert.

6. Medizinisches Implantat gemäß einem der vorherigen Ansprüche, **dadurch gekennzeichnet, dass** die Platten (21, 31) über Aussparungen (413), Stege (423) oder Einschnitte (433) verfügen, die dazu geeignet sind die Steifigkeit des Implantats zu reduzieren.

7. Medizinisches Implantat gemäß einem der vorherigen Ansprüche, **dadurch gekennzeichnet, dass** die Platten (21, 31) an zu einer Deckfläche (101) orientierten Stirnseiten eine Verzahnung (212, 412, 422, 432) enthalten, deren Kontur so gestaltet ist, dass sich ein Hinterschnitt ergibt.

8. Medizinisches Implantat gemäß einem der vorherigen Ansprüche, **dadurch gekennzeichnet, dass** die Platten (21, 31) an zu einer Deckfläche (101) orientierten Stirnseiten eine Verzahnung (212, 412, 422, 432) enthalten, deren Zähne einzeln oder segmentweise über geeignete Aussparungen (413, 423, 433) in den Platten (21, 31) elastisch federnd bzw. nachgiebig gelagert sind.

9. Medizinisches Implantat gemäß einem der vorherigen Ansprüche, **dadurch gekennzeichnet, dass** die Aussparungen (213) semipermeabel sind oder eine Vielzahl von Bohrungen bzw. Poren enthält, die Aufgrund ihres Durchmessers zur Zelldifferentiation oder zum Aufbau eines osmotischen Drucks geeignet sind.

10. Medizinisches Implantat gemäß einem der vorherigen Ansprüche, **dadurch gekennzeichnet, dass** die Platten (21, 32, 501, 502) über Verbindungselemente oder einen Stoff- oder Formschluss mit mindestens einer Aufnahme (5050) verbunden sind, und die Aufnahme (5050) mit mindestens einer Aussparung (571) versehen ist, die zur Aufnahme eines Knochenankers geeignet ist.

11. Medizinisches Implantat gemäß einem der vorherigen Ansprüche, **dadurch gekennzeichnet, dass** eine Materialstärke der Platten zwischen 0,2 und 1,5mm beträgt und/oder ein Abstand (22) zwischen den Platten weniger als 1,5mm beträgt.

## Claims

1. Medical implant (2) for placing between bones, in particular between vertebral bodies, wherein the implant consists of a multiplicity (>3) of thin plates (21, 31), the material thickness of the plates lies between 0.02 and 3 mm, the plates are provided with recesses (213), which are suitable for body cells to populate and grow through, the plates are connected by at least one connecting element (23, 32), the plates are arranged largely parallel to one another in at least one direction, **characterised in that** the spacing (22) between the plates lies between 0.02 mm and 3 mm.

2. Medical implant according to claim 1, **characterised in that** the connecting element (23, 5051, 5053) is in the form of a screw, pin or tube.

3. Medical implant according to any of the preceding claims, **characterised in that** the spacing (22) between the plates (21) is defined by rings (5020) or discs, which are provided on the connecting element (23, 5051, 5052, 5053).

4. Medical implant according to any of the preceding claims, **characterised in that** the spacing (22) of the plates (21) is defined by embossing or mouldings.

5. Medical implant according to any of the preceding claims, **characterised in that** the connecting element (32) is in the form of a strip or plate, the connecting element (32) has slits (311), and the spacing of the slits (311) defines the spacing (22) of the plates (31).

6. Medical implant according to any of the preceding claims, **characterised in that** the plates (21, 31) have recesses (413), webs (423) or notches (433) which are suitable for reducing the rigidity of the implant.

7. Medical implant according to any of the preceding claims, **characterised in that** the plates (21, 31) contain, on end faces oriented towards a top surface (101), a tooth system (212, 412, 422, 432) which has a contour so shaped as to produce an undercut.

8. Medical implant according to any of the preceding claims, **characterised in that** the plates (21, 31) contain, on end faces oriented towards a top surface (101), a tooth system (212, 412, 422, 432) with teeth which are mounted, elastically sprung or flexible, individually or in segments, via suitable recesses (413, 423, 433) in the plates (21, 31).

9. Medical implant according to any of the preceding claims, **characterised in that** the recesses (213) are semi-permeable or contain a multiplicity of drilled holes or pores which, on account of their diameter, are suitable for cell differentiation or for the build-up of an osmotic pressure.

10. Medical implant according to any of the preceding claims, **characterised in that** the plates (21, 32, 501, 502) are connected via connecting elements or an adhesive bond or by form closure to at least one seat (5050), and the seat (5050) is provided with at least one recess (571) which is suitable for accommodating a bone anchor.

11. Medical implant according to any of the preceding claims, **characterised in that** a material thickness of the plates lies between 0.2 and 1.5 mm and/or the spacing (22) between the plates is less than 1.5 mm.

## Revendications

1. Implant médical (2) destiné à être placé entre des os, en particulier entre des corps vertébraux, dans lequel l'implant consiste en une pluralité (>3) de plaques minces (21, 31), une épaisseur de matériau des plaques est entre 0,02 et 3 mm, les plaques sont dotées d'évidements (213), qui sont adaptés pour être colonisés et comblés par des cellules somatiques, les plaques sont reliées par au moins un élément de raccordement (23, 32), les plaques sont agencées de manière essentiellement parallèle l'une à l'autre dans au moins une direction, **caractérisé en ce qu'**une distance (22) entre les plaques est entre 0,02 mm et 3 mm.

2. Implant médical selon la revendication 1, **caractérisé en ce que** l'élément de raccordement (23, 5051, 5053) est réalisé en tant que vis, tige ou tube.

3. Implant médical selon l'une quelconque des revendications précédentes, **caractérisé en ce qu'**une distance (22) est définie entre les plaques (21) par des anneaux (5020) ou disques, lesquels se trouvent sur les éléments de raccordement (23, 5051, 5052, 5053).

4. Implant médical selon l'une quelconque des revendications précédentes, **caractérisé en ce que** la distance (22) entre les plaques (21) est définie par des empreintes ou formations décalées.

5. Implant médical selon l'une quelconque des revendications précédentes, **caractérisé en ce que** l'élément de raccordement (32) est réalisé sous la forme d'une bande ou plaque, l'élément de raccordement (32) dispose de fentes (311) et la distance entre les fentes (311) définit la distance (22) entre les plaques (31).

6. Implant médical selon l'une quelconque des revendications précédentes, **caractérisé en ce que** les plaques (21, 31) disposent d'évidements (413), d'âmes (423) ou d'incisions (433) qui sont adaptés pour réduire la rigidité de l'implant.

7. Implant médical selon l'une quelconque des revendications précédentes, **caractérisé en ce que** les plaques (21, 31) comprennent une denture (212, 412, 422, 432) au niveau de côtés frontaux orientés vers une surface de recouvrement (101) dont le contour est réalisé de sorte qu'il en résulte une contre-dépouille.

8. Implant médical selon l'une quelconque des revendications précédentes, **caractérisé en ce que** les plaques (21, 31) comprennent une denture (212, 412, 422, 432) au niveau de côtés frontaux orientés vers une surface de recouvrement (101) dont les dents sont logées de manière élastique ou souple individuellement ou par segment par le biais d'évidements (413, 423, 433) dans les plaques (21, 31).

9. Implant médical selon l'une quelconque des revendications précédentes, **caractérisé en ce que** les évidements (213) sont semi-perméables ou comprennent une pluralité de perçages ou de pores, qui sont adaptés en raison de leur diamètre à la différenciation cellulaire ou à la formation d'une pression osmotique.

10. Implant médical selon l'une quelconque des revendications précédentes, **caractérisé en ce que** les plaques (21, 32, 501, 502) sont reliées à au moins un logement (5050) par le biais d'éléments de raccordement ou une correspondance de matière et de forme, et le logement (5050) est prévu avec au moins un évidement (571) qui est adapté pour le logement d'un ancrage osseux.

11. Implant médical selon l'une quelconque des revendications précédentes, **caractérisé en ce qu'**une épaisseur de matériau entre les plaques est entre 0,2 et 1,5 mm et/ou une distance (22) entre les plaques est inférieure à 1,5 mm.
